Europäisches Patentamt

European Patent Office

Office européen des brevets

(11) Publication number: **0 289 669 B1**

# EUROPEAN PATENT SPECIFICATION

(45) Date of publication of patent specification: **01.04.92**

(51) Int. Cl.5: **C07C 47/575**, C07C 45/64, C07C 45/59, A01N 35/04

(21) Application number: **87304008.3**

(22) Date of filing: **05.05.87**

The file contains technical information submitted after the application was filed and not included in this specification

(54) **Process for preparing 3-phenoxybenzaldehydes.**

(43) Date of publication of application:
**09.11.88 Bulletin 88/45**

(45) Publication of the grant of the patent:
**01.04.92 Bulletin 92/14**

(84) Designated Contracting States:
**BE CH DE FR IT LI NL SE**

(56) References cited:
**EP-A- 0 051 235
GB-A- 1 415 945
GB-A- 1 543 964
GB-A- 2 055 799
GB-A- 2 185 016**

**HOUBEN-WEYL "Methoden der Organischen Chemie", 4th edition, vol. V, part 3, 1965, pages 85-89, Georg Thieme Press, Stuttgart;**

(73) Proprietor: **HARDWICKE CHEMICAL COMPANY
451 Florida Boulevard
Baton Rouge Louisiana 70801(US)**

(72) Inventor: **Hardwicke III, James Ernest
708 Arbutus Drive
Columbia South Carolina 29205(US)**

(74) Representative: **Bizley, Richard Edward et al
BOULT, WADE & TENNANT 27 Furnival Street
London EC4A 1PO(GB)**

## Description

This invention relates to a process for preparing 3-phenoxybenzaldehydes.

As disclosed in British Patents 1,539,733 (Sheldon et al.) and 2,055,799 (Thiault et al.), it is known that 3-phenoxybenzaldehydes are useful as pesticide intermediates and that they can be prepared from 3-bromobenzaldehyde acetals. Sheldon et al., who react their acetals with phenols or alkali metal phenolates in the presence of a copper catalyst, require an aprotic organic solvent for their reaction. Thiault et al. avoid the need for the aprotic solvent by reacting their acetal with an excess of phenol in the presence of a copper catalyst and potassium carbonate at a temperature of 170-210°C.

Although the process of Thiault et al. is advantageous in that it avoids several problems associated with the use of the solvents of Sheldon et al., it also has its disadvantages. The elevated temperatures required for the reaction lead to degradation of the acetal, and the potassium carbonate appears to poison the catalyst. Thus, the obtainable yield of product is comparatively low.

An object of this invention is to provide a novel process for preparing 3-phenoxybenzaldehydes and acetal intermediates therefor.

Another object is to provide such a process that requires no aprotic organic solvent.

A further object is to provide such a process that can be conducted at moderate temperatures.

These and other objects are attained by reacting a 3-bromobenzaldehyde acetal with an alkali metal phenolate at a temperature of 130-165°C. in the presence of a copper catalyst and a phenol as the sole solvent to prepare a 3-phenoxybenzaldehyde acetal and, if desired, hydrolyzing the resultant acetal with an acid to form a 3-phenoxybenzaldehyde.

3-Bromobenzaldehyde acetals (bromoacetals) that can be used in the practice of the invention are the acetals derived from 3-bromobenzaldehydes and dihydric alcohols, such as ethylene glycol. The 3-bromobenzaldehyde may be substituted or unsubstituted, any substitutents being inert substitutents (i.e., substituents that will not prevent the reaction from occuring), such as alkyl, aryl, and alkoxy groups containing 1-10 carbons. A preferred 3-bromobenzaldehyde acetal is 3-bromobenzaldehyde acetal itself.

The alkali metal phenolate that is reacted with the bromoacetal is an alkali metal salt of phenol or a substituted phenol bearing one or more inert substituents, such as the optional substituents on the bromobenzaldehyde. The preferred phenolates are sodium and potassium phenolates, with the potassium phenolates being particularly preferred in the interest of driving the reaction to completion. However, when it is desired to use a sodium salt, complete reaction can be achieved by employing it in conjunction with a small amount of potassium salt, e.g., about 10% of the total salt. The most preferred phenolates are generally the salts of phenol itself. The phenolate is preferably employed in excess, e.g., in a phenolate/bromoacetal mol ratio of at least 1.1/1, optimally 1.12/1, to drive the reaction to completion. Except for the practical limitations of economics and the size of the reaction vessel, there does not appear to be any maximum to the amount of phenolate that may be used.

The phenol employed as a solvent, like the phenolic moiety of the phenolate, may be substituted or unsubstituted phenol, and it is most practically the phenol corresponding to the phenolic moiety of the phenolate. It is generally employed in a phenol/phenolate mol ratio of at least 0.14/1, most commonly 0.5/1. Higher mol ratios, e.g., 1/1 are apt to be preferred as minimum mol ratios when the phenolate is a sodium salt; and even higher mol ratios can be employed if desired, regardless of the nature of the phenolate, since there does not appear to be any maximum to the amount of phenol that may be used. However, it is generally preferred to avoid using too large an excess of the phenol because of the larger reaction vessel that would be required to accomodate it.

The copper catalyst may be a cuprous or cupric compound, such as a chloride, bromide, iodide, fluoride, oxide, p-chlorobenzoate, and phenolate, for example. However, cupric or cuprous chloride is generally preferred. It is used in catalytic amounts, generally 0.001-0.1 mol per mol of bromoacetal.

The reaction is conducted by mixing the bromoacetal, phenolate, phenol, and copper catalyst and heating the mixture to 130-165°C., preferably 140-150°C., to effect conversion of the bromoacetal to the corresponding phenoxyacetal. Temperatures substantially above 165°C. should be avoided because of increased tar formation as the temperature is increased, and temperatures below about 130°C. are undesirable because of the reduced reaction rate at lower temperatures. The time required for the reaction varies with factors like the particular temperature employed but is frequently in the range of 2-4 hours.

In a preferred embodiment of the invention, the reaction is conducted as the final step of a total reaction that comprises (1) reacting the 3-bromoacetaldehyde with a dihydric alcohol by conventional means to prepare the bromoacetal, (2) separately reacting the phenol with an alkali metal hydroxide in amounts such as to provide the particular amounts of phenol and phenolate desired in the coupling reaction mixture, the reaction being conducted in a suitable solvent, such as xylene, at a suitable temperature, such as reflux temperature, and the water of reaction and the solvent being removed during the reaction and/or after the phenolate has been formed, and (3) adding the copper catalyst to the phenolate/phenol mixture formed in step 2, adding the bromoacetal formed in step 1 gradually or as a single charge, and heating the resultant mixture at 130-165° C.

After completion of the reaction, the phenoxyacetal may be converted to the corresponding 3-phenoxybenzaldehyde by conventional means. Typically, the phenoxyacetal is hydrolyzed with hydrochloric acid, although, as mentioned by Thiault et al, other strong acids are also utilizable.

The invention is particularly advantageous as a means of preparing 3-phenoxybenzaldehydes and their acetal intermediates under moderate conditions while avoiding the problems inherent in the use of aprotic organic solvents. The moderate temperatures permitted by the absence of potassium carbonate minimize tar formation and minimize reduction of the bromoacetal during the coupling reaction.

The following example is given to illustrate the invention and is not intended as a limitation thereof.

EXAMPLE

Part A

A suitable reaction vessel was charged with one molar proportion of 3-bromobenzaldehyde, 1.46 molar proportions or ethylene glycol, and 0.002 molar proportion of toluene/xylene sulfonic acid and warmed to 155° C. Water formed by the reaction was allowed to distill out and collect in a receiver. When the rate slowed, vacuum was applied to strip out the remaining water and the excess ethylene glycol. Conversion to 3-bromobenzaldehyde acetal was 98-99%.

Part B

A suitable reaction vessel was charged with 0.2 molar proportion of xylene, 1.82 molar proportions of phenol, and 1.12 molar proportions of KOH. The mixture was heated until about 1.5 molar proportions of water were collected. The potassium phenolate was cooled below reflux and one molar proportion of the 3-bromobenzaldehyde acetal of Part A was added. The temperature was adjusted to 130-135° C. and the xylene solvent was stripped off under vacuum.

Part C

Nitrogen was applied to the reaction mixture of Part B to break the vacuum, and 0.002 molar proportion of cuprous chloride was added. The reaction was stirred for 3.5-4 hours at 130-145° C. and cooled to 90° C. About 11 molar proportions of water were added, and the pH was adjusted to 6 with dilute HCl. GC analysis of the organic layer showed a 99% conversion of the starting bromoacetal with a 98% selectivity to 3-phenoxybenzaldehyde acetal.

Part D

KBr was drained off from the crude product of Part C, which was then heated under vacuum to strip out the excess phenol. The crude product was then hydrolyzed with about 0.06 molar production of HCl added as 2.5% HCl at 90-100° C. for 30 minutes three times. The resulting 3-phenoxybenzaldehyde was then distilled at 140-145° C. best vacuum. The yield, based on 3-bromobenzaldehyde, was about 85%.

3-Phenoxybenzaldehydes made by the process of the present invention may be used in pesticidal formulations.

## Claims

1. A process for preparing a 3-phenoxybenzaldehyde acetal by reacting a 3-bromobenzaldehyde acetal with an alkali metal phenolate in the presence of a copper catalyst at a temperature of 130-165° C in the presence of a phenol as the sole solvent.

2. A process for preparing a 3-phenoxybenzaldehyde which process comprises reacting a 3-bromobenzaldehyde acetal with an alkali metal phenolate in the presence of a copper catalyst at a temperature of 130-165° C in the presence of a phenol as the sole solvent to form a 3-bromobenzaldehyde acetal which is thereafter hydrolysed with an acid to form a 3-phenoxybenzaldehyde.

3. A process as claimed in claim 1 or claim 2 in which the alkali metal phenolate is sodium of potassium phenolate or a mixture thereof.

4. A process as claimed in claim 3 in which the alkali metal phenolate is potassium phenolate.

5. A process as claimed in claim 1 or claim 2 in which the copper catalyst is either cuprous chloride or cupric chloride.

## Revendications

1. Procédé de préparation d'un acétal de 3-phénoxybenzaldéhyde par la réaction d'un acétal de 3-bromobenzaldéhyde avec un phénolate de métal alcalin en présence d'un catalyseur de cuivre à une température de 130-165°C en présence d'un phénol comme seul solvant.

2. Procédé de préparation d'un 3-phénoxybenzaldéhyde, qui comprend la réaction d'un acétal de 3-bromobenzaldéhyde avec un phénolate de métal alcalin en présence d'un catalyseur de cuivre à une température de 130-165°C en présence d'un phénol comme seul solvant pour former un acétal de 3-bromobenzaldéhyde qui est ensuite hydrolysé avec un acide pour former un 3-phénoxybenzaldéhyde.

3. Procédé suivant l'une ou l'autre des revendications 1 et 2, caractérisé en ce que le phénolate de métal alcalin est du phénolate de sodium ou de potassium ou un mélange de ceux-ci.

4. Procédé suivant la revendication 3, caractérisé en ce que le phénolate de métal alcalin est le phénolate de potassium.

5. Procédé suivant l'une ou l'autre des revendications 1 et 2, caractérisé en ce que le catalyseur de cuivre est du chlorure cuivreux ou du chlorure cuprique.

**Patentansprüche**

1. Verfahren zur Herstellung eines 3-Phenoxybenzaldehydacetals durch Reaktion eines 3-Brombenzaldehydacetals mit einem Alkalimetallphenolat in Gegenwart eines Kupferkatalysators bei einer Temperatur von 130-165°C in Gegenwart eines Phenols als dem einzigen Lösungsmittel.

2. Verfahren zur Herstellung eines 3-Phenoxybenzaldehyds, wobei das Verfahren die Reaktion eines 3-Brombenzaldehydacetals mit einem Alkalimetallphenolat in Gegenwart eines Kupferkatalysators bei einer Temperatur von 130-165°C in Gegenwart eines Phenols als einzigem Lösungsmittel umfaßt, um ein 3-Phenoxybenzaldehydacetal zu bilden, das anschließend mit einer Säure zur Bildung eines 3-Phenoxybenzaldehyds hydrolysiert wird.

3. Verfahren nach Anspruch 1 oder 2, worin das Alkalimetallphenolat Natrium- oder Kaliumphenolat oder ein Genmisch davon ist.

4. Verfahren nach Anspruch 3, worin das Alkalimetallphenolat Kaliumphenolat ist.

5. Verfahren nach Anspruch 1 oder 2, worin der Kupferkatalysator entweder Kupfer-(I)-chlorid oder Kupfer-(II)-chlorid ist.